(19) **Europäisches Patentamt / European Patent Office / Office européen des brevets**

(11) **EP 2 388 030 B2**

## (12) NEW EUROPEAN PATENT SPECIFICATION
After opposition procedure

(45) Date of publication and mention
of the opposition decision:
**16.03.2022 Bulletin 2022/11**

(45) Mention of the grant of the patent:
**07.08.2013 Bulletin 2013/32**

(21) Application number: **10005282.8**

(22) Date of filing: **20.05.2010**

(51) International Patent Classification (IPC):
***A61M 1/16*** (2006.01)    ***A61M 1/36*** (2006.01)

(52) Cooperative Patent Classification (CPC):
**A61M 1/16; A61M 1/1601; A61M 1/3609;**
A61M 2205/502; A61M 2205/505; A61M 2230/65

(54) **Kidney substitution device to automate blood sampling procedure in a kidney substitution treatment machine**

Nierenersatzvorrichtung zur Standardisierung und/oder Automatisierung eines Blutprobenahmeverfahrens in einer Nierenersatzbehandlungsmaschine

Dispositif de remplacement de rein pour normaliser et/ou automatiser la procédure d'échantillonnage de sang dans une machine de traitement de remplacement de rein

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(43) Date of publication of application:
**23.11.2011 Bulletin 2011/47**

(73) Proprietor: **B. Braun Avitum AG**
**34212 Melsungen (DE)**

(72) Inventors:
• **Castellarnau, Alex**
**34212 Melsungen (DE)**
• **Kuhl, Martin**
**36251 Bad Hersfeld (DE)**

(74) Representative: **Winter, Brandl - Partnerschaft mbB**
**Alois-Steinecker-Straße 22**
**85354 Freising (DE)**

(56) References cited:
**US-A- 5 954 951    US-A1- 2010 051 551**

**Description**

The invention relates to a method and a device to standardize the blood sampling procedure in a kidney substitution treatment machine. Said standardization maximizes the accuracy of blood based Kt/V measurements, while it enhances the usability of the kidney substitution treatment machine by relieving the user of paying attention to the standard blood sampling procedure.

Dialysis adequacy is the topic that has got and gets more attention when one thinks about patient outcome. In order to estimate dialysis adequacy one needs a parameter establishing a relation between dialysis dosage and patient outcome. The most accepted parameter to estimate the quantity of dialysis delivered or dosage is the Kt/V, where K is the effective clearance for urea, t is the treatment time and V is the urea distribution volume which matches the total body water.

The NCDS (National Cooperative Dialysis Study) and the HEMO study found, after analyzing a large patient group, that morbidity and mortality in end stage renal disease (ESRD) was strongly correlated with the Kt/V value or dialysis dose. Data obtained from these studies resulted in guidelines regarding hemodialysis treatments, which demand a minimum dose of Kt/V=1.2 for non-diabetic patients and 1.4 for diabetics (DOQI guidelines). It is worthy to point out that a morbidity decrease not only improves the patient well-being, but also reduces significantly the medical costs as the patient requires less care.

Two main methods are available to estimate the hemodialysis dose: the formal urea kinetic model (UKM) and two-pool model. (European Best Practice Guidelines for Hemodialysis; Section II: Hemodialysis Adequacy).

The UKM assumes single pool kinetics with volume contraction over the treatment time. This model makes a computer based iterative estimation of V and urea generation rate (G). It provides reliable and simultaneous information about the efficiency of the HD session in terms of single pool Kt/V (spKt/V); the nutritional status of the patient in terms of normalized protein catabolic rate (nPCR); and an individualized prescription of the treatment time necessary to achieve a given Kt/V level.

The calculation procedure requires a dialyzer clearance (K) value and three blood samples: pre- and post-HD blood urea for the first session of the week and pre-HD blood urea for the second session of the week. The K value can be derived from the dialyzer mass transfer-area coefficient (KoA) or from conductivity measurements. (Depner T, Garred L. Solute transport mechanisms in dialysis. Hörl W, Koch K, Lindsay R, Ronco C, Winchester JF, editors. Replacement of renal function by dialysis, 5th ed. Kluwer academic publishers, 2004:73-91 ).

The major drawback of the UKM is its single pool nature. It does not consider the physiological urea compartmentalization. The blood flow delivered to different tissues is unequal. Thus, during HD, urea sequestration occurs in tissues that are relatively poor perfused like muscle and skin. Once the dialysis is complete a slow 30-60 minutes re-equilibration between high and low perfused areas takes place.

The lack of consideration of the urea rebound introduces an over-estimation to the equilibrated Kt/V value, which typically lies between 10% and 17% in standard HD sessions, but can be up to 45% in some patients after high efficiency treatments.

Cell model and Regional Blood Flow (RBF) model have been proposed to explain the two-pool kinetics followed by the urea and other solutes during a dialysis treatment. In the RBF model, the unequal perfusion of different body tissues is responsible for the urea rebound. While the RBF model is good to explain small size molecules kinetics, it is not good for other compounds affected by the transport characteristics across membranes, like for example phosphate or $\beta$2-Microglobulin. These compounds are better modelled by the cell model, where the effect of limited solute permeability of the cell membrane causes a concentration disequilibrium between intracellular and extracellular space.

The mathematical complexity of two-pool kinetics restricts its use to experimental studies. However simplified equations approximating the equilibrated Kt/V (eKt/V) have been developed by Daugirdas. First, the Daugirdas 2nd generation equation (E1) delivers a spKt/V, which accounts for volume contraction and urea generation over the treatment time. Five inputs are required: urea concentration before (C0) and after the dialysis (Ct), treatment time (T), ultrafiltration (UF) and dry weight (W):

$$spKt/V = -\ln\left(\frac{C_t}{C_0} - 0.008 \cdot \frac{T}{60}\right) + \left(4 - 3.5 \cdot \frac{C_t}{C_0}\right) \cdot \frac{UF}{W} \quad (E1)$$

Further, the rate equation express the Kt/V in terms of eKt/V by using the spKt/V and the treatment time as inputs. Two expressions are available, one for arteriovenous access (E2) and one for venovenous access (E3):

$$eKt/V = spKt/V - \frac{0.6}{(T/60)} \cdot spKt/V + 0.03 \quad \text{(E2)}$$

$$eKt/V = spKt/V - \frac{0.47}{(T/60)} \cdot spKt/V + 0.02 \quad \text{(E3)}$$

Daugirdas equations are widely used and have been accepted as the standard method to express the adequacy of a dialysis treatment. (Daugirdas JT. Simplified equations for monitoring Kt/V, PCRn, eKt/V, and ePCRn. Adv Ren Replace Ther. 1995 Oct ;2(4):295-304.).

The accuracy of the method is strongly influenced by the protocol followed to obtain the blood samples, specially important is the sampling time of the post dialysis sample. The National Kidney Foundation (NKF), describes in the "Guideline 3. Methods for Postdialysis Blood Sampling" of the Kidney Disease Outcomes Quality Initiative Guidelines (KDOQI), a standard blood sampling protocol aimed to maximize the method accuracy.

A transcription of said guideline follows:

*"When dialysis adequacy is assessed by using predialysis and postdialysis blood urea nitrogen (BUN) measurements, the following considerations should be accounted:*

- *Both samples (predialysis and postdialysis) should be drawn during the same treatment session.*
- *The risk of underestimating predialysis BUN level because of saline dilution or by sampling the blood after treatment has begun should be avoided.*
- *The risk of underestimating the postdialysis BUN level because of access recirculation (AR) should be avoided by first slowing the blood flow through the dialyzer to a rate at which AR is expected to be minimal (100 mL/min) for a period long enough to ensure that unrecirculated blood has advanced to below the sampling port (usually 15 seconds).*
- *An alternative method is to stop the dialysate flow for a period long enough to increase the dialysate outlet BUN level close to that of the blood inlet BUN level (3 minutes) before obtaining the postdialysis sample.*

*Slow-Blood-Flow Method for Obtaining the Postdialysis Sample*

*A. Drawing the sample from the bloodline sampling port*

*At the completion of HD, turn off the dialysate flow and decrease the UFR to 50 mL/hr, to the lowest TMP/UFR setting, or off. If the dialysis machine does not allow for turning off the dialysate flow, or if doing so violates clinic policy, decrease the dialysis flow to its minimal setting.*

*Decrease the blood flow to 100 mL/min for 15 seconds (longer if the bloodline volume to the sampling port exceeds 15 mL). To prevent pump shut-off as the blood flow rate is reduced, it may be necessary to manually adjust the venous pressure limits downward. At this point, proceed to obtain your sample. You can either shut off the blood pump before sampling, or leave it running at 100 mL/min while the sample is being drawn. After the sample has been obtained, stop the blood pump (if not already stopped) and complete the patient disconnection procedure as per dialysis clinic protocol.*

*B. Method that avoids use of an exposed needle: Drawing the sample from the arterial needle tubing using a syringe or vacutainer device.*

*Proceed with steps (1) and (2) as per A above.*

*After the 15 second slow-flow period (a slow flow period is still required to clear the small volume in the arterial needle tubing of recirculated blood), stop the pump. Clamp the arterial and venous blood lines. Clamp the arterial needle tubing. Disconnect the blood line tubing from the inlet bloodline, and attach either a syringe or a Vacutainer with a Leur-Lok type connection to the arterial needle tubing (or arterial port of the venous catheter). Release the clamp on the arterial needle tubing and obtain the blood sample.*

*Proceed with step (3) as in section A above.*

*Stop-Dialysis-Flow Method of obtaining the Postdialysis Sample*

*At the completion of HD, turn off the dialysate flow (or put it into bypass) and decrease the UFR to 50 mL/hr, to the lowest TMP/URF setting, or off.*

*Wait 3 minutes. Do NOT reduce the blood flow rate during this 3-min period. Obtain the blood sample, either from the sample port on the inlet bloodline, or from the arterial needle tubing or from the arterial port of the venous catheter if using the needle-free method as described in the table above, part B. If sampling from the inlet bloodline, it does not matter if you stop or do not stop the blood flow while this sample is being taken. It probably is best to stop the blood pump prior to sampling. In the stop-dialysate-flow method, slowing the blood flow prior to sampling should not be done. After the sample has been obtained, return the patient's blood in the bloodlines and dialyzer per protocol. "*

The European Best Practice Guidelines for Haemodialysis also describes a standard blood sampling protocol in the "Guideline II.4: Monitoring of treatment". A transcription of said guideline follows:

*"The procedure that should be used to obtain a post-dialysis blood sample follows:*

- *Set UF rate to zero.*
- *Decrease the blood flow to 100 ml/umin for 15 s. This is the best approximated time necessary for new, not recirculated blood to rinse the tubing between needle and arterial sampling port.*
- *Exactly after 15 s draw the blood sample from the arterial sampling port nearest to the patient. In this case the effect of cardiopulmonary recirculation is still present, and the first of the two equations reported in Guideline II.1.2 must be used to calculate eKtuV.*

*The final sample may be drawn 1-2 min after slowing the blood pump, when the arteriovenous urea gradient due to the cardiopulmonary effect has dissipated This method has the advantage to avoid the variability of the cardiopulmonary effect, but is associated with the risk of underestimating the spKtuV, due to the very early effect of intercompartmental urea re-equilibration. "*

Our clinical experience has demonstrated that obtaining and accurate post-dialysis sample is not easy. Even with trained medical staff there's always a major or minor degree of deviation from the standard protocol. These deviations impact the accuracy of the final eKt/V value.

A hemodialysis setup of the prior art is described in a patent of a "reagent supply for a hemodialysis system" (US 2010/0051551 A1): the hemodialysis setup encloses a portable hemodialysis unit, where the hemodialysis unit includes suitable components for performing hemodialysis including a dialyzer, one or more pumps to circulate blood through the dialyzer, a source of dialysate, and one or more pumps to circulate the dialysate through the dialyzer. The hemodialysis setup includes a housing that supports the components of the hemodialysis unit and has a front panel at which blood circuit connections and dialysate fluidic connections are located. For example, the front panel supports blood line connections for patient blood access, connections for a reagent supply, dialyzer connections for both blood flow and dialysate, etc. The reagent supply arrangement for the hemodialysis system includes an E-prong connector having three parallel prongs with two outer prongs arranged in a common plane and a center prong arranged above the common plane, a first supply line for a first reagent connected in fluid communication with one of the outer prongs, a second supply line for a second reagent connected in fluid communication with the other of the outer prongs, a liquid line connected in fluid communication with the center prong, and a container for housing the first reagent having an inlet connected to the liquid line and an outlet connected to the first supply line for the first reagent. The E-prong connector may help prevent the improper connection to the dialysis unit, e.g., because the prong arrangement ensures connection in only one way to the dialysis unit. Nevertheless, the disclosed setup is lacking an accurate post-dialysis sampling operation. As a whole, the system only comprises a blood sample port (19), where a standardized blood-sampling has not been disclosed.

The problem of this invention is to provide a reliable kidney substitution treatment device to improve the usability of the kidney substitution treatment device by automating and/or standardizing the post-dialysis sampling procedure as much as possible.

This problem is solved by a device with the features described in claim 1. Further developments of the invention are described in the claims 2 and 3.

The kidney substitution treatment device with the features of claim 1 is aimed to both enhance the usability of the device and to minimize the errors bound to the post-dialysis sampling by controlling the human factor. Said standardization and/or automating maximizes the accuracy of blood based Kt/V measurements, while it enhances the usability of the kidney substitution treatment machine by relieving the user of paying attention to the standard blood sampling procedure.

In a first embodiment of the invention the kidney substitution treatment can be double needle hemodialysis, single needle hemodialysis, single needle cross over hemodialysis, post-dilution hemodiafiltration, pre-dilution hemodiafiltration, pre-post-dilution hemodiafiltration, post-dilution hemofiltration, pre-dilution hemofiltration, pre-post-dilution hemofiltration or sequential hemodialysis so that the invention is usable in devices for all known kidney substitution treatment.

In a further advantageously embodiment of the invention the actuating means is

a) a key on the user interface which has to be pressed ors switched or
b) a field on a display of the user interface which has to be clicked by clicking means like a computer mouse
c) a field on a touch screen of the user interface which has to be pressed
d) an order to start the routine of actions at at least one pre defined time after starting the device or at a pre defined time after shut down the device.

With all this alternatives of the actuating means which should not be understand as a final listing a easy and secure starting of an automated and/or standardized blood sampling is possible for the user without any need to supervise the blood sampling procedure anymore.

In a not-claimed aspect, it has been found very useful that the routine of actions triggered by the actuating means is as follows:

- turn off the dialysate flow or set the machine in bypass mode,
- keep the same blood flow, which was set when triggered the routine of actions,
- starts a 3 minutes timer, and
- when the 3 minutes are over generating a signal or a message telling or showing that a blood sample may be taken from the arterial sampling port.

As a further not-claimed aspect, a useful routine of actions triggered by the actuating means is as follows:

- turn off the dialysate flow or set the machine in bypass mode,
- keep the same blood flow, which was set when triggered the routine of actions,
- starts a 3 minutes timer, and
- when the 3 minutes are over stop the blood pump, and
- generating a signal or a message telling or showing that the arterial line can be disconnected and that the blood sample may be taken.

As a further not-claimed aspect, the routine of actions triggered by the actuating means is as follows:

- set UF rate to 0,
- decrease the blood flow to 100 ml/min,
- start a 1 to 2 minutes timer,
- when the time is over generating a signal or a message telling or showing that the blood sample may be taken from the arterial sampling port is shown to the user.

As a further not-claimed aspect,, the routine of actions triggered by the actuating means is as follows:

- sets UF rate to 0,
- decrease the blood flow to 100 ml/min,
- start a 1 to 2 minutes timer,
- when the time is over stop the blood pump,
- generating a signal or a message telling or showing that the arterial line can be disconnected and that the blood sample may be taken.

The routine of actions according to the invention triggers by the actuating means warrants a secure and easy automated and/or standardized blood sampling without any need for the user to supervise the blood sampling procedure anymore.

Advantageously, the routine of actions according to the not-claimed aspects as the aforementioned listed are implemented in the device and are selectable by a user and/or by a technician via a user interface.

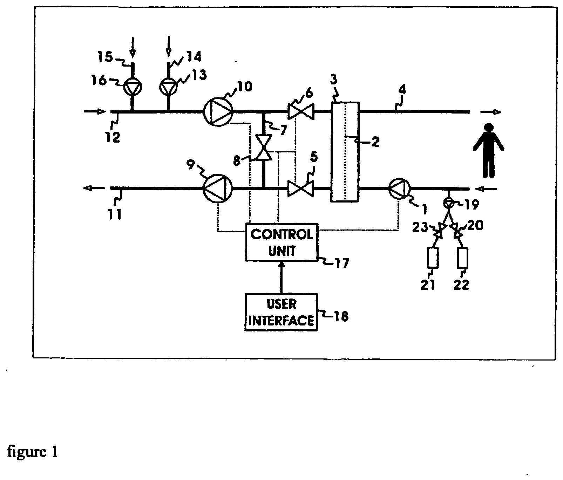

FIG 1 Depicts a schematic view of a conventional dialysis machine where the invention can be used.

Figure 1 shows a draw of the dialysate circuit of a conventional dialysis machine where the present invention can be applied. The dialysis machine provides means for replacing the renal function of a mammal if the renal function is impaired or completely absent. The blood from the patient is pumped into the blood chamber of the dialyzer 3 by the blood pump 1 and returns to the patient through the tube 4. Through the semipermeable membrane 2 the patient blood is cleared of uremic substances after contacting with fresh dialysis solution, which is produced online by the dialysis machine.

The dialysis fluid is prepared online by the dialysis machine from water and one or several concentrates. The machine depicted in Fig. 1 comprises a water inlet 12, concentrate inlets 14 and 15, and concentrate pumps 13 and 16.

A first main pump 10 forces the fresh dialysis fluid into the dialysate chamber of the dialyzer 3, where the dialysis fluid clears the patient blood of uremic compounds. A second main pump 9 sucks the spent dialysate and any ultrafiltrate coming from the patient out of the dialyzer 3.

A by pass line 7 and the valves 5, 6 and 8 provide means to control the flow path of the dialysis fluid.

The pump 19, the valves 23 and 20 and the blood sampling chambers 21 and 22 provide means to automate the blood sampling process.

The control unit 17 acts as an interface between the user and the disclosed devices. The control unit 17 receives commands introduced by the user on the user interface 18 and actuates on the disclosed devices.

Under normal condition both valves 23 and 20 are closed. The user interface 18 has a blood sampling key, when pressed after connecting the patient and before starting the therapy, the valve 20 is opened and the blood pump 19 draws a pre-dialysis blood sample which is stored on the blood sample chamber 22. When the blood sampling key is pressed after the end of the therapy and before disconnecting the patient, the control unit 17 takes the necessary measures to set the conditions for obtaining an optimal post-dialysis blood sample (see standard protocol described above), the valve 23 is opened and the blood pump 19 draws a post-dialysis blood sample which is stored on the blood sample chamber 21.

The blood sample chambers have single-use disposables which are sent to the lab for urea concentration analysis. It is also possible to include means to directly analyze the urea concentration within the blood sample chambers 21 and 22.

In our preferred embodiment the machine shows on the touch screen a blood sampling key. When touched or clicked, the necessary sequence of events to obtain a standard blood sample are triggered. The key can be placed not only on the touch screen but in any position of the dialysis machine housing.

A configuration screen in the machine technical interface allows the technician to set which protocol has to be followed by the blood sampling feature: Slow-Blood-Flow method, Stop-Dialysis-Flow method or Cardiopulmonary recirculation method. When the Slow-Blood-Flow method is selected, an extra configuration option allows to adjust the dialysate flow: either off or the minimum which is allowed by the dialysis centre policy.

An additional configuration option sets whether to draw the blood sample from the bloodline sampling port or from the arterial needle tubing using a syringe or vacutainer device. The later avoids the use of an exposed needle.

The machine will trigger different events according to the technical mode settings:

If Slow-Blood-Flow method and bloodline sampling port are selected, by clicking the blood sampling key, the machine performs automatically the following actions:

- Turns off the dialysate flow or reduces it to the minimum which is allowed by the dialysis centre policy. (can be set in technical model)
- Decreases the blood flow to 100 ml/min.
- Starts a 15 seconds timer.
- When the time is over a message telling that the postdialysis blood sample may be taken from the arterial sampling port is shown to the user.

If Slow-Blood-Flow method and arterial needle tubing sampling are selected, by clicking the blood sampling key, the machine performs automatically the following actions:

- Turns off the dialysate flow or reduces it to the minimum which is allowed by the dialysis centre policy. (can be set in technical model)
- Decreases the blood flow to 100 ml/min.
- Starts a 15 seconds timer.
- Stops the blood pump.
- A message notifies the user that the arterial line can be disconnected and that the postdialysis blood sample may be taken.

The following described alternative is not claimed:

If Stop-Dialysis-Flow method and bloodline sampling port are selected, by clicking the blood sampling key, the machine performs automatically the following actions:

- Turns off the dialysate flow or sets the machine in bypass mode.

- Keeps the same blood flow.
- Starts a 3 minutes timer.
- When the time is over a message telling that the postdialysis blood sample may be taken from the arterial sampling port is shown to the user.

The following described alternative is not claimed:
If Stop-Dialysis-Flow method and arterial needle tubing sampling are selected, by clicking the blood sampling key, the machine performs automatically the following actions:

- Turns off the dialysate flow or sets the machine in bypass mode.
- Keeps the same blood flow.
- Starts a 3 minutes timer.
- Stops the blood pump.
- A message notifies the user that the arterial line can be disconnected and that the postdialysis blood sample may be taken.

The following described alternative is not claimed:
If Cardiopulmonary recirculation method and bloodline sampling port are selected, by clicking the blood sampling key, the machine performs automatically the following actions:

- Sets UF rate to 0.
- Decreases the blood flow to 100 ml/min.
- Starts a 1 to 2 minutes timer.
- When the time is over a message telling that the postdialysis blood sample may be taken from the arterial sampling port is shown to the user.

The following described alternative is not claimed:
If Cardiopulmonary recirculation method and arterial needle tubing sampling are selected, by clicking the blood sampling key, the machine performs automatically the following actions:

- Sets UF rate to 0.
- Decreases the blood flow to 100 ml/min.
- Starts a 1 to 2 minutes timer.
- Stops the blood pump.
- A message notifies the user that the arterial line can be disconnected and that the postdialysis blood sample may be taken.

Reference signs

1 -pump
2 - semi-permeable membrane
3 - dialyzer
3a - blood chamber
3b - fluid chamber
4 - extra corporeal blood system
5 - valve
6 - valve
7 - pass line
8 - valve
9 -pump
10 - pump
11 - dialyzing fluid/water outlet
12 - dialyzing fluid/water inlet
11, 12 - dialyzing fluid system
13 - pump
14 - concentrate inlet

15 - concentrate inlet
16 - pump
17 - control unit
18 - user interface
19 - pump
20 - valve
21 - blood sampling chamber
22 - blood sampling chamber
23 - valve

**Claims**

1. A kidney substitution treatment device

   wherein the device has an extracorporeal blood system (4) which is connected with a blood chamber (3a) of a dialyzer (3), which is divided by a semi-permeable membrane (2) into the blood chamber (3a) and a dialyzing fluid chamber (3b) and wherein the device has a dialyzing fluid system (11, 12) which is connected to the dialyzing fluid chamber (3b) of the dialyzer (3)
   wherein the extracorporeal blood system (4) has a pump (19), at least one valve (23,20) which divides the extracorporeal blood system (4) from at least one blood sampling chambers (21,22)
   **characterized in that**
   the device has a user interface (18) which communicates with a control unit (17) wherein the user interface (18) has actuating means to trigger a routine of actions which is stored on a storage device which communicates with the control unit (17) wherein that routine comprises:

   a) an order to turn off the dialysate flow through the dialyzer (3) or to reduce the dialysate flow through the dialyzer (3) to a minimum which is allowed by the dialysis centre policy and
   b) an order to decrease the blood flow to a range between 0 and 150 ml/min and
   c) an order to run the device with the dialysate flow through the dialyzer (3) defined in paragraph a) and a blood flow defined in paragraph b) for a pre defined time by timer and
   d) an order to generate a signal or a message that a blood sample may be taken from an arterial sampling port or that the arterial line can be disconnected and that the blood sample may be taken when the pre defined time of paragraph c) has lapsed.

2. The device according to claim 1, wherein the kidney substitution treatment can be double needle hemodialysis, single needle hemodialysis, single needle cross over hemodialysis, post-dilution hemodiafiltration, pre-dilution hemodiafiltration, pre-post-dilution hemodiafiltration, post-dilution hemofiltration, pre-dilution hemofiltration, prepost-dilution hemofiltration or sequential hemodialysis.

3. The device according to claim 1 or 2, wherein the actuating means is

   a) a key on the user interface (18) which has to be pressed or switched or
   b) a field on a display of the user interface (18) which has to be clicked by clicking means like a computer mouse
   c) a field on a touch screen of the user interface (18) which has to be pressed
   d) an order to start the routine of actions at at least one pre defined time after starting the device or at a pre defined time after shut down the device.

**Patentansprüche**

1. Vorrichtung zur Nierenersatzbehandlung,

   wobei die Vorrichtung über ein extrakorporales Blutsystem (4) verfügt, welches mit einer blutseitigen Kammer (3a) eines Dialysators (3) verbunden ist, der durch eine semipermeable Membran (2) in die blutseitige Kammer (3a) und eine dialysatseitige Kammer (3b) geteilt ist, und wobei die Vorrichtung über ein Dialyseflüssigkeitssystem (11, 12) verfügt, welches mit der dialysatseitigen Kammer (3b) des Dialysators (3) verbunden ist, wobei das extrakorporale Blutsystem (4) eine Pumpe (19), mindestens ein Ventil (23, 20) aufweist, welches das extrakorporale Blutsystem (4) von mindestens einer Blutsammelkammer (21, 22) abtrennt,
**dadurch gekennzeichnet, dass**
die Vorrichtung eine Bedienoberfläche (18) aufweist, welche mit einer Steuereinheit (17) kommuniziert, wobei die Bedienoberfläche (18) ein Betätigungsmittel hat, um ein Handlungsprogramm einzuleiten, welches in einem Speicher abgelegt ist, der mit der Steuereinheit (17) kommuniziert, wobei das Programm umfasst:

a) einen Befehl um den Dialysatfluss durch den Dialysator (3) abzuschalten oder den Dialysatfluss durch den Dialysator (3) auf ein von den Dialysezentrumsrichtlinien erlaubtes Minimum zu verringern und
b) einen Befehl um die Blutflussrate in einen Bereich zwischen 0 und 150 ml/min abzusenken und
c) einen Befehl um die Vorrichtung mit dem Dialysatfluss durch den Dialysator (3), der im Absatz a) festgesetzt wurde, und mit dem Blutfluss, der im Ansatz b) festgesetzt wurde, über einen durch einen Timer zuvor festgelegten Zeitraum zu betreiben und
d) einen Befehl um ein Signal oder eine Mitteilung zu erzeugen, dass eine Blutprobe aus einem arteriellen Probenanschluss entnommen werden kann oder dass das arterielle Leitungssystem entkoppelt werden kann und dass die Blutprobe entnommen werden kann, wenn der zuvor festgelegte Zeitraum aus Abschnitt c) abgelaufen ist.

2. Vorrichtung nach Anspruch 1, wobei die Nierenersatzbehandlung eine Doppel-Nadel-Hämodialyse, eine Einzel-Nadel-Hämodialyse, eine Einzel-Nadel-Cross-Over-Hämodialyse, eine Post-Dilutions-Hämodiafiltration, eine Pre-Dilutions-Hämodiafiltration, eine Pre-Post-Dilutions-Hämodiafiltration, eine Post-Dilutions-Hämofiltration, eine Pre-Post-Dilutions-Hämofiltration oder eine sequentielle Hämodialyse sein kann.

3. Vorrichtung nach Anspruch 1 oder 2, wobei das Betätigungsmittel Folgendes ist

a) eine Taste auf der Bedienoberfläche (18), die gedrückt oder geschaltet werden muss, oder
b) ein Feld auf einem Display der Bedienoberfläche (18), das mit einem Klickmittel wie einer Computermaus angeklickt werden muss,
c) ein Feld auf einem Touchscreen der Bedienoberfläche (18), das gedrückt werden muss 5,
d) ein Befehl zum Starten des Handlungsprogramms zu mindestens einer vordefinierten Zeit nach dem Starten der Vorrichtung oder zu einer vordefinierten Zeit nach dem Abschalten der Vorrichtung.

**Revendications**

1. Dispositif de traitement de substitution rénal

dans lequel le dispositif présente un système sanguin extracorporel (4) qui est branché à un compartiment de sang (3a) d'un dialyseur (3) qui est divisé par une membrane semi-perméable (2) en le compartiment de sang (3a) et un compartiment de fluide à dialyse (3b) et dans lequel le dispositif présente un système de fluide à dialyse (11, 12) qui est relié au compartiment de fluide à dialyse (3b) du dialyseur (3)
dans lequel le système sanguin extracorporel (4) présente une pompe (19), au moins un clapet (23, 20) qui divise le système sanguin extracorporel (4) depuis au moins un compartiment de prélèvement sanguin (21, 22)
**caractérisé en ce que**
le dispositif présente une interface utilisateur (18) qui communique avec une unité de commande (17), dans lequel l'interface utilisateur (18) présente un moyen d'actionnement pour déclencher une routine d'actions qui est stockée sur un dispositif de stockage qui communique avec l'unité de commande (17), dans lequel la routine comprend :

a) une instruction pour faire cesser le flux de dialysat à travers le dialyseur (3) ou pour réduire le flux de dialysat à travers le dialyseur (3) à un minimum qui est autorisé par le règlement du centre de dialyse et
b) une instruction pour réduire le flux de dialysat à une plage entre 0 et 150 ml/min et
c) une instruction pour faire fonctionner le dispositif avec le flux de dialysat à travers le dialyseur (3) défini au paragraphe a) et un flux de dialysat défini au paragraphe b) pendant un temps prédéfini par une minuterie et
d) une instruction pour produire un signal ou un message qui indique qu'un prélèvement sanguin peut être effectué depuis un point de prélèvement artériel ou qu'un cathéter artériel peut être débranché et que le prélèvement sanguin peut être effectué lorsque le temps prédéfini du paragraphe c) s'est écoulé.

**2.** Dispositif selon la revendication 1, dans lequel le traitement de substitution rénal peut être une hémodialyse à aiguille double, une hémodialyse à aiguille unique, une hémodialyse croisée à aiguille unique, une hémodiafiltration en post-dilution, une hémodiafiltration en pré-dilution, une hémodiafiltration en pré-post-dilution, une hémofiltration en post-dilution, une hémofiltration en pré-dilution, une homofiltration en pré-post-dilution ou une hémodialyse séquentielle.

**3.** Dispositif selon les revendications 1 ou 2, dans lequel le moyen d'actionnement est

a) une touche sur l'interface utilisateur (18) qui doit être enfoncée ou commutée ou

b) un champ sur un afficheur de l'interface utilisateur (18) qui doit être cliqué par un moyen de cliquage telle une souris d'ordinateur

c) un champ sur un écran tactile de l'interface utilisateur (18) qui doit être enfoncé

d) une instruction pour lancer la routine d'actions à au moins un temps prédéfini après le démarrage du dispositif ou à un temps prédéfini après l'arrêt du dispositif.

figure 1

**EP 2 388 030 B2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100051551 A1

**Non-patent literature cited in the description**

- Solute transport mechanisms in dialysis. **DEPNER T ; GARRED L.** Replacement of renal function by dialysis. Kluwer academic publishers, 2004, 73-91

- **DAUGIRDAS JT.** Simplified equations for monitoring Kt/V, PCRn, eKt/V, and ePCRn. *Adv Ren Replace Ther,* October 1995, vol. 2 (4), 295-304